## Europäisches Patentamt

## European Patent Office

## Office européen des brevets

(19)

(11) Publication number: **0 100 234**
**B1**

(12) **EUROPEAN PATENT SPECIFICATION**

(45) Date of publication of patent specification: **01.06.88**

(51) Int. Cl.⁴: **G 01 N 29/04,** G 01 N 29/00

(21) Application number: **83304306.0**

(22) Date of filing: **26.07.83**

(54) **Ultrasonic measurement of characteristic values of a medium.**

(30) Priority: **26.07.82 JP 129902/82**

(43) Date of publication of application:
**08.02.84 Bulletin 84/06**

(45) Publication of the grant of the patent:
**01.06.88 Bulletin 88/22**

(84) Designated Contracting States:
**DE FR GB NL SE**

(56) References cited:
**EP-A-0 064 399**
**US-A-3 896 411**
**US-A-4 047 172**

(73) Proprietor: **FUJITSU LIMITED**
**1015, Kamikodanaka Nakahara-ku**
**Kawasaki-shi Kanagawa 211 (JP)**

(72) Inventor: **Miwa, Hirohide c/o FUJITSU LIMITED**
**Patent Department 1015, Kamikodanaka**
**Nakahara-ku Kawasaki-shi Kanagawa 211 (JP)**
Inventor: **Ueda, Mitsuhiro c/o FUJITSU LIMITED**
**Patent Department 1015, Kamikodanaka**
**Nakahara-ku Kwaasaki-shi Kanagawa 211 (JP)**

(74) Representative: **Sunderland, James Harry et al**
**HASELTINE LAKE & CO Hazlitt House 28**
**Southampton Buildings Chancery Lane**
**London WC2A 1AT (GB)**

**The file contains technical information submitted after the application was filed and not included in this specification**

Courier Press, Leamington Spa, England.

**Description**

This invention relates to ultrasonic measurement of characteristic values, such as $\beta$ (attenuation slope), of a medium under investigation, for example living body tissue.

As an ultrasonic wave travels through a transmission medium, for example living body tissue, it suffers attenuation. An attenuation constant, indicating the degree of attenuation suffered by the ultrasonic wave as it travels through the transmission medium, is proportional to the frequency f of the wave. The value of the constant of proportionality—$\beta$, called attenuation slope—is characteristic of the medium or tissue involved.

Moreover, a reflection coefficient for an ultrasonic wave in a medium is proportional to wave frequency raised to a power n, and the power exponent n is also characteristic of the medium or tissue involved.

The inventors of the invention the subject of this application have determined that, theoretically, a reflection coefficient can be expressed in the form:—

$$bf^4 e^{-(df)^2}$$

Here, b and d have values characteristic of the tissue or medium concerned.

However, on the basis of the theoretical expression of reflection coefficient as a function of frequency f, as explained above, there has been no method generally established for obtaining living body tissue characteristics from a power spectrum profile.

The inventors of the invention the subject of the present application have proposed a method of obtaining values of n and $\beta$, for characterising a medium or living body tissue, using energy ratios, taking the above-mentioned function to be a power n function of frequency f.

That method is effective but involves consideration of at least three quantities relating to energy over a narrow bandwidth. For this reason, errors can easily arise as a result of local unevennesses in the energy spectrum, which unevennesses are said to result in spectrum scalloping. Accordingly, the method has disadvantages in that calculations are necessary for each different possible pair of quantities which can be constituted from the three quantities mentioned above relating to three frequencies in an effective frequency band, and in that statistical processings must be effected for aggregation or averaging of obtained values of n and $\beta$, and as a result many calculations are required.

According to the present invention there is provided:—

A method of measuring characteristics of a medium under investigation, excluding any method for treatment of the human or animal body by surgery or therapy and any diagnostic method practised on the human or animal body, in which measuring method ultrasonic signals are transmitted into the medium and reflected utlrasonic signals are received by an ultrasonic signal sending and receiving system, and characteristics of the medium are measured by analysing the received signals, the measuring method comprising the following steps:

(a) frequency-analyzing received signals reflected from a region of the medium, the characteristics of which region are to be measured, to obtain the power spectrum of those signals,

(b) normalizing the power spectrum obtained in step (a) with a power spectrum of the sending and receiving system per se, to obtain the transfer function R(f) of the medium under investigation for the received signals reflected from the said region,

(c) normalizing R(f) with a value R(fo) thereof relating to a particular frequency fo, to obtain a function P(f),

(d) obtaining P(f)/A(f), where A(f) is a term in a function Q(f) obtained by normalizing a theoretically or empirically estimated transfer function of the medium for signals reflected from the said region with a value of the theoretically or empirically estimated transfer function relating to the particular frequency fo, which function Q(f) has the form

$$Q(f) = A(f) \cdot e^{B(f)},$$

where A(f) and B(f) include parameters the values of which are characteristic of the said region of the medium,

(e) expressing P(f)/A(f) obtained in step (d) as a logarithmic exponent, and

(f) obtaining values of medium-characteristic parameters included in the terms A(f) and B(f) by calculation, e.g. by regression processing with the exponent obtained in step (e), as a function of frequency, taken to be equal to the term B(f) of the theoretically or empirically estimated transfer function.

The invention also includes an apparatus as set out in claim 7.

In an embodiment of the present invention A(f) in the step d is expressed by an equation which is proportional to power n (n is a constant indicating a medium characteristic) of the frequency f, B(f) is indicated by a linear function of frequency f, and the linear coefficient of frequency f is related to the line integration of the frequency slope (attenuation slope) of the attenuation constant of the medium.

In an embodiment of the present invention B(f) in the step d is expressed by the quadratic equation in frequency f, and the coefficients of the constant, linear and quadratic terms are respectively dependent upon average micro-miniature structure, line integration of attenuation slope and self-correlation distance in the scanning direction.

**0 100 234**

An embodiment of this invention, for obtaining a transfer function of a medium under investigation, for example a living body, from received signals corresponding to ultrasonic waves reflected in the medium, and for obtaining for example living body tissue characteristics therefrom, can provide:

1) that a spectrum shape is obtained avoiding problems caused by discontinuous changes at interfaces between different regions of the medium under investigation,

2) that easy regression from measured values is possible using functions derived theoretically or empirically expressing spectrum shape as a product of an exponential function and a non-exponential function, and

3) that parameters involved in such functions are determinable by such regression and moreover that characteristic values of the medium under investigation are obtained.

In an embodiment of the present invention, the frequency response (power-frequency) spectrum of a living body tissue transfer function is normalized and only the shape of the spectrum is considered. Absolute values of the spectrum are not considered. Moreover, using functions estimated from theory or experiment involving the product of an exponential function and a non-exponential function, parameters relating to characteristics of the medium under investigation (e.g. living body tissue) contained in the exponential part of the exponential function can be obtained through regression from measured values, using a logarithmic operation after division of the above-mentioned product by the non-exponential function.

Where attenuation constant is proportional to frequency (constant of proportionality $\beta$) and reflection coefficient is a function of frequency determined by various characteristic values of the medium under investigation, an embodiment of this invention provides for measurement of the characteristic values. The characteristic values are obtained by regression from a theoretical or empirical equation using measured power spectrum values.

Reference is made, by way of example, to the accompanying drawings, in which:—

Figure 1 is a schematic cross-sectional view illustrating living body tissue structure along a Z axis ultrasonic wave scanning direction,

Figure 2 is a schematic block diagram of apparatus for putting an embodiment of this invention into effect,

Figure 3 is a waveform diagram showing waveforms occurring at points in the apparatus of Figure 2, and

Figure 4 shows graphs illustrating frequency characteristics (power-frequency spectrums) for assistance in understanding the present invention.

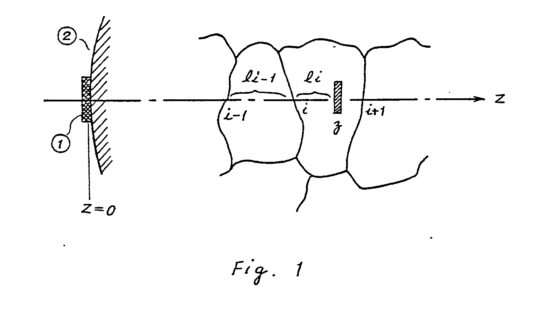

It will be assumed for the purposes of explanation that ultrasonic wave pulses of a center frequency fc and of a bandwidth $2\Omega$ are transmitted from a transducer 1 as shown in Figure 1 into a deep region of a living body through the surface of the body (in a direction $+Z$), that the pulse waveforms travel to each location within the living body at a sound velocity C, that reflected waves are sequentially transmitted in the reverse direction $(-Z)$ at the sound velocity C and that these reflected waves are received by the transducer 1.

It will also be assumed that there are to be measured the characteristics of tissue at a depth z in the living body.

As shown, the living body has a variety of regions of different kinds of tissue through its depth. These regions are labelled i−1, i i+1 in Figure 1. Sound velocity C is almost constant through these regions.

A sound pulse transmitted from the surface of living body 2, that is, from the position z=0, suffers attenuation. The attenuation constant of each region i for sound of a frequency f is proportional to the frequency f. Indicating the attenuation constant of region i also by the letter i, and the constant of proportionality for region i as $\beta i$:—

$$i = ai + \beta i \cdot f \quad (ai \text{ is a constant})$$

$\beta i$ is a parameter which is characteristic of the kind of tissue in region i and is called attenuation slope or attenuation inclination.

In each region, it indicates a power reflection coefficient r(f) which is a function of frequency.

At the interface between two regions, acoustic impedance changes significantly and the surface at the interface is often smooth in a living body. Thus, when the transmitted sound pulse passes from one region to another, e.g. from region i−1 to region i, a small transmission loss occurs. As a result, as the transmitted pulse travels from region to region, there is a step by step transmission loss. Transmissivity, from region i−1 to region i is indicated by $\tau i$.

In the same way, a reflected wave caused by reflection of a transmitted sound pulse by the living tissue passes from region i to region i−1 and suffers a loss. In this case transmissivity is indicated by $\tau' i$.

$\tau i$ and $\tau' i$ are considered to have no frequency dependency.

After transmission of a sound pulse, the transducer 1 continuously receives reflected waves from successively deeper regions. Since a reflected wave corresponding to the depth z (i.e. reflected at depth z) will be received at a time t=2z/C after pulse transmission, tissue characteristics for tissue at depth z can be obtained by analyzing the received reflected waveform occurring over a certain narrow time width proximate to the above time $t$.

3

A power spectrum $Er(f)$ of a received signal reflected at the depth $z$ can be easily obtained using a known frequency analyzer such as an FFT device (Fast-Fourier-Transformer).

The power spectrum $Er(f)$ depends on the square of a variety of factors:— the frequency characteristic of the transducer employed, the frequency characteristic of the converging beam at the depth $z$, the transfer function of other parts of the measuring system and the transfer function of living body tissue, resulting from transmission and reflection within a living body.

With a standard reflector placed at the depth $z$ in water, for example, a power spectrum $Eo(f)$ of the received wave can be obtained. $Eo(f)$ then shows the square of transfer function of the measuring system (i.e. $Eo(f)$ contains all the factors mentioned above except for the transfer function of the living body tissue). When $Er(f)$ is divided by $Eo(f)$ and normalized, the square of intrinsic transfer function of a living body tissue is obtained.

Now, below, an actually measured power spectrum of the transfer function of a living body tissue will be indicated as $R(f)$.

On the other hand, from above explanation, the power spectrum is theoretically expressed by the following equation:—

$$\overline{R(f)} = k \cdot r(f) \cdot \left( \prod_{i=0}^{Z} \tau i \cdot \tau'i \right) \exp\left( -4 \sum_{i=0}^{Z} \beta i f l i \right) \tag{1}$$

where

$k$ is a constant not depending on $f$,

$li$ is the distance within the region $i$ over which sound travels (on its way to and from a zone of a desired depth in the body).

It will be understood that the symbol $\pi$ means accumulation, namely

$$\prod_{i=0}^{n} Ai = A_0 \times A_1 \times A_2 \times \ldots \times A_n.$$

It will also be understood that $r(f)$ is the reflection coefficient of the tissue in the zone of the desired depth in the body, and that $Z$ is the number of the region in which that zone falls.

Explained below is a method of obtaining tissue characteristics by comparing $R(f)$ and $\overline{R(f)}$.

One transducer is capable only of obtaining $R(f)$ for a frequency range within its effective bandwidth. $R(f)$ over a wider frequency range can be obtained by obtaining and combining respective $R(f)$'s relating to respective frequency ranges within the wider range using a plurality of transducers.

In actual measurement, the power spectrum has local unevennesses arising from interference of reflected waves from adjacent reflecting tissues and other causes, and exhibits so-called spectrum scalloping. As a result there can occur significant errors in measurement of power spectrum shape.

In order to prevent such errors, it is preferable to make measurements in respect of adjacent regions in a measuring area, for example, front and back, left and right, or upper and lower regions thereof, or to make measurements in respect of these regions several times and then to execute static processings such as equalization etc., from the viewpoint of time and space.

Equation (1) can be standardized (normalized) as indicated below in equation (2) by using $\overline{R(fo)}$ relating to a particularly frequency $fo$ (for example, a frequency $fm$ which gives the maximum value of equation (1) etc.) in equation (1).

$$Q(f) = \overline{R(f)}/\overline{R(fo)} = \frac{r(f)}{r(fo)} \times \frac{\exp\{-(4\Sigma\beta i l i)f\}}{\exp\{-4(\Sigma\beta i l i)fo\}} \tag{2}$$

It will be seen that factors $k$, $\tau i$, $\tau'i$ are eliminated in equation (2). Elimination of the unknown factors $\tau i$ and $\tau'i$ obtains a significant advantage.

Since $fo$ and $R(fo)$ can be obtained as the actual measured values, an actual measured power spectrum can also be standardized (normalized) by using $R(fo)$ as indicated below.

$$P(f) = R(f)/R(fo) \tag{3}$$

Therefore, parameters involved in $Q(f)$ can be determined by regression (working backwards), without any need to take into account $\tau i$ and $\tau'i$, under the condition that $P(f)$ is equal to $Q(f)$.

The function $r(f)$ can be expressed in various forms according to approximate values therefore. Explained below are an experimental (empirical) form:—

$$r(f) = a \cdot f^n \tag{4}$$

(a: constant; n: constant dependent upon the kind of tissue involved), and a theoretical equation form provided by the inventors of the present invention:—

$$r(f) = b \cdot b' \cdot f^4 \cdot \exp\{-\sigma^2_z \cdot (\frac{2\pi}{C})^2 \cdot f^2\} \tag{5}$$

where,

b′ is a function of frequency depending on transducer size and radius of curvature (known),

b is a constant depending on average micro-miniature structure of the kind of tissue involved,

$\sigma_z$ is a self-correlational distance on space in the utlrasonic wave sending/receiving direction of tissue, and

C is sound velocity.

Equations (4) and (5) appear to differ significantly in form, but in a practically used frequency range provide values which approximately match.

When equations (4) and (5) are substituted into equation (2), $Q_4$ and $Q_5$ are respectively obtained, as follows:—

$$Q_4 = A_4 f^n \cdot \exp\{-4(\Sigma\beta ili)f\} \tag{6}$$

$$Q_5 = A_5 b' f^4 \exp\{-4(\Sigma\beta ili)f - (\frac{2\pi\sigma_z}{C})^2 f^2\} \tag{7}$$

Where,

$$A_4 = (a/R(fo)) \cdot \prod_{i=0}^{z} (\tau_i \cdot \tau_i') \cdot R$$

$$A_5 = (b/R(fo)) \cdot \prod_{i=0}^{z} (\tau_i \cdot \tau_i') \cdot R$$

As will be understood from equations (6) and (7), it is a characteristic of both that $Q$ ($Q_4$, $Q_5$) is given as a product of an exponential function and a non-exponential function. Dividing each equation by its non-exponential function and taking logarithms gives:—

$$\ln Q_4/A_4 - n\ln f = -4(\Sigma\beta ili) \cdot f \tag{8}$$

$$\ln Q_5/A_5 - \ln b'f^4 = -4(\Sigma\beta ili)f - (\frac{2\pi\sigma_z}{C})^2 f^2 \tag{9}$$

Here, with an actually measured P substituted in place of $Q_4$ and $Q_5$ the left sides of equations (8) and (9) can be calculated with regression so that these equations can be established with maximum accuracy. Thereby, the parameters n, $\Sigma\beta i$ li, $\sigma_z$ can be obtained.

Equation (8) contains an unknown value n on its left side, but a value of n which conforms to the requirement that the right side of the equation be a linear function of f can be determined by changing it variously (by taking measurement at different values of f).

A value of $\beta i$ of each tissue in Figure 1 can be obtained from a difference by obtaining a value of $\Sigma\beta ili$ at the depth z. When $\beta$ is expressed as a continuous function of z, its line integration is expressed as $\int_0^z \beta \, dz$.

Therefore, a value of $\beta(z)$ can be obtained by differentiation or can be obtained from the line integration in each direction by the algorithm of X-ray CT (Computer Tomography).

Values of n, $\beta$, $\sigma_z$ can be obtained on a scanning line of the one measuring direction, and a distribution picture of n, $\beta$ and $\sigma_z$ in a plane can be obtained by scanning this scanning line in said plane.

Above, the principle employed in an embodiment of the present invention is explained. In actual measurement, it is widely executed to extract parameters from the sampling points at a certain depth z by Doppler measurement. The extracted waveforms are Fourier-analyzed and means known as a DFT (Digital Fourier Transformer) are used easily as the means for obtaining the power spectrum of such waveforms. An output can be input to a computer and all subsequent calculations can be executed by programs.

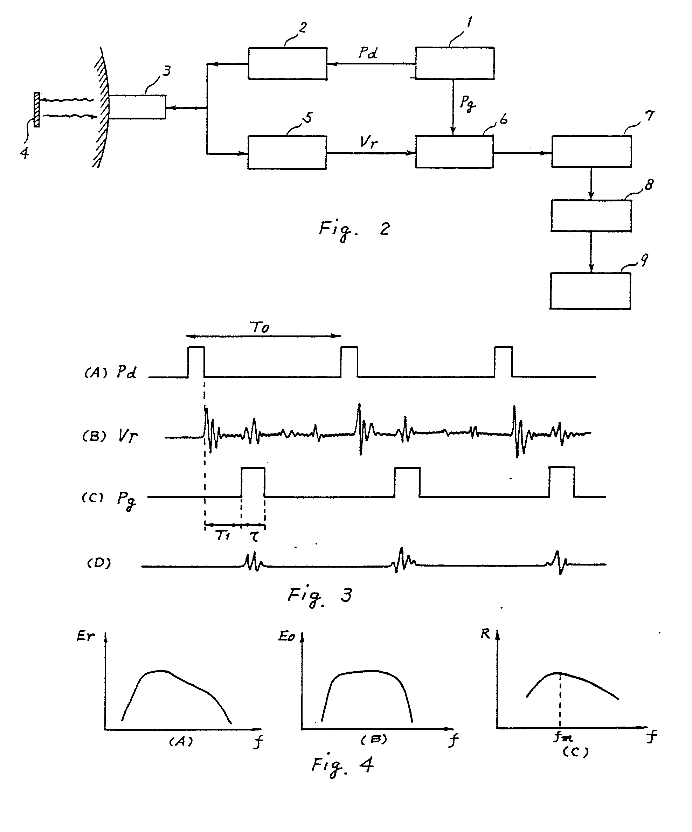

The general form of a system for putting an embodiment of the present invention into effect will be described with reference to Figure 2.

A synchronisation signal Pd, for synchronising sending of ultrasonic waves, as shown in waveform (A) of Figure 3, is transmitted to a sending circuit 2 from a timing control circuit 1, and a transducer 3 is driven by a pulse from sending circuit 2 having a power sufficient for transducer 3 to transmit an ultrasonic wave. Thereby an ultrasonic wave is transmitted into a living body (or into a medium to a standard reflector).

A reflected wave, reflected from living body tissue (or from a standard reflector) 4, is received by the transducer 3, and is amplified to a suitable level by a receiving circuit 5. The amplified signal is then sent to a data collecting circuit 6 as a received signal Vr shown in waveform (B) of Figure 3.

The timing control circuit 1 sends a gate pulse Pg, as shown in waveform (C) of Figure 3, to the data collecting circuit 6 at a timing as delayed by a period T1 from synchronization signal Pd, the delay T1 corresponding to the distance from the surface of the transducer to a zone in the living tissue (or to the standard reflector) in respect of which measurements are to be made. Thereby reflected signals from the desired measuring region are forwarded as data.

The width $\tau$ of Pg is determined to correspond to the range to be measured. The data collected and forwarded is shown by waveform (D) in Figure 3.

The collected data is forwarded to a frequency analyzer 7 and the result of frequency analysis is sent to a data memory 8. Frequency analysis obtains as a result the reflected wave spectrum. An example of a reflected wave spectrum from living body tissue is shown in graph (A) in Figure 4. A reflected wave spectrum from a standard reflector is shown in graph (B) in Figure 4.

An arithmetic operation circuit 9 performs various calculations on the results of frequency analysis stored in a data memory 8, in accordance with the procedures described above, and obtains a desired result.

More specifically, the spectrum shown in graph (A) in Figure 4 is normalized by the spectrum shown in graph (B). Thereby a spectrum (corresponding to equation (1) above) as shown in graph (C) in Figure 4 is obtained. Thus, $f_0$ of equation (3) can be obtained, and moreover the left sides of the equations (8) and (9) calculated. Thereafter, calculation by regression is carried out and values of n, $\Sigma\beta i l i$ and $\sigma$ are determined. A value of $\beta i$ can also be obtained from $\Sigma\beta i l i$ for each depth z.

The arithmetic operation circuit 9 may have any structure or form so long as it can carry out the necessary calculations. For example, a micro-computer consisting of a micro-processor, RAM, ROM, I/O port etc. may be used.

Apparatus as shown in Figure 2, operating in accordance with an embodiment of this invention can provide that:—

1) effects of noncontinuous transmission at an interface between different tissue regions can be eliminated by extracting through normalization a living body tissue characteristic function not influenced by the measuring system used and by normalizing again against the value of the function with at a certain frequency,

2) regression calculation can be realized easily by dividing an estimated function by a non-exponential function and introducing logarithmic expressions, and

3) tissue characteristics can be obtained from parameters obtained as provided by 2).

Although the present invention has been described particularly in the context of the measurement of characteristics of living body tissues, it will be understood that the invention can be used for measuring characteristics of other mediums.

An embodiment of this invention provides a reflection type ultrasonic living body tissue characterization method capable of obtaining highly accurate measuring results. Reflected ultrasonic wave power from the tissue under investigation is measured for respective frequency ranges. Then, it is normalized using the result of measurement of reflected ultrasonic wave power for a standard reflector placed in a region corresponding to the region to be measured in the tissue. Thereby, characteristics of the measuring system can be eliminated. Moreover, unknown values not dependent on frequency are eliminated by normalizing measured values relating to other frequencies with a measured value relating to a particular frequency. In addition, the corresponding theoretical equation is expressed by a simple arithmetic equation by indicating the normalized values using a logarithmic expression. Thereafter, various parameters indicating tissue characteristics are obtained by regressing calculations.

### Claims

1. A method of measuring characteristics of a medium (2) under investigation excluding any method for treatment of the human or animal body by surgery or therapy and any diagnostic method practised on the human or animal body, in which measuring method ultrasonic signals are transmitted into the medium and reflected ultrasonic signals are received by an ultrasonic signal sending and receiving system (1; 3, 2, 5), and characteristics of the medium are measured by analysing the received signals, the measuring method comprising the following steps:

(a) frequency-analyzing received signals reflected from a region (i) of the medium (2), the characteristics of which region are to be measured, to obtain the power spectrum of those signals,

(b) normalizing the power spectrum obtained in step (a) with a power spectrum of the sending and receiving system per se to obtain the transfer function R(f) of the medium under investigation for the received signals reflected from the said region,

(c) normalizing R(f) with a value R(fo) thereof relating to a particular frequency fo, to obtain a function P(f),

(d) obtaining P(f)/A(f), where A(f) is a term in a function Q(f) obtained by normalizing a theoretically or empirically estimated transfer function of the medium for signals reflected from the said region with a

6

value of the theoretically or empirically estimated transfer function relating to the particular frequency fo, which function Q(f) has the form

$$Q(f) = A(f) \cdot e^{B(f)},$$

where A(f) and B(f) include parameters the values of which are characteristic of the said region of the medium,

(e) expressing P(f)/A(f) obtained in step (d) as a logarithmic exponent, and

(f) obtaining values of medium-characteristic parameters included in the terms A(f) and B(f) by calculation, e.g. by regression processing with the exponent obtained in step (e), as a function of frequency, taken to be equal to the term B(f) of the theoretically or empirically estimated transfer function.

2. A measuring method as claimed in claim 1, wherein A(f) is a function of a power n where n is a constant, the value of which is characteristic of the said region, and of frequency f, and wherein B(f) is a linear function of frequency f, the coefficient of frequency f in the function being related to the line integration of the attenuation slopes of the attenuation constants of regions of the medium through which signals reflected from the said region pass.

3. A measuring method as claimed in claim 1, wherein B(f) is a quadratic function of frequency f, in which the coefficients of the term linear in f and the term quadratic in f are respectively related to the line integration of the attenuation slopes of the regions of the medium through which signals reflected from the said region pass, and the self-correlation distance in the scanning direction.

4. A measuring method as claimed in claim 3, wherein A(f) is a function of the fourth power of frequency f.

5. A measuring method as claimed in any preceding claim, wherein the power spectrum of the sending and receiving system per se is obtained using a standard reflector in a disposition, in relation to the system, corresponding to the disposition of the said region of the medium under investigation.

6. A measuring method as claimed in any preceding claim wherein the medium is living body tissue.

7. Apparatus for measuring characteristics of a medium under investigation, including

an ultrasonic signal sending and receiving system (2, 3, 5), operable to transmit ultrasonic signals into the medium and to receive reflected ultrasonic signals from the medium, and

means (1, 6, 7, 8, 9) for analyzing the received signals to measure characteristics of the medium, comprising:—

frequency-analyzer means (7), operable to frequency analyze the received signals reflected from a region of the medium, the characteristics of which region are to be measured, to obtain the power spectrum of those signals, and

means (8, 9) operable to normalize the power spectrum with a power spectrum of the sending and receiving system (2, 3, 5) per se, to obtain the transfer function R(f) of the medium under investigation for the received signals reflected from the said region, and operable to

normalize R(f) with a value R(fo) thereof relating to a particular frequency fo, to obtain a function P(f), and operable to

obtain P(f)/A(f) by dividing P(f) by A(f), where A(f) is a term in a function Q(f) obtained by normalizing a theoretically or empirically estimated transfer function of the medium for signals reflected from the said region with a value of the theoretically or empirically estimated transfer function relating to the particular frequency fo, which function Q(f) has the form

$$Q(f) = A(f) \cdot e^{B(f)},$$

where A(f) and B(f) include parameters the values of which are characteristic of the said region of the medium, and operable to

express P(f)/A(f) as a logarithmic exponent, and operable to

obtain values of medium-characteristic parameters included in the terms A(f) and B(f) by calculation, e.g. by regression processing with the said logarithmic exponent, as a function of frequency, taken to be equal to the term B(f) of the theoretically or empirically estimated transfer function.

**Patentansprüche**

1. Verfahren zur Messung von Charakteristiken eines zu untersuchenden Mediums (2), ausschließlich irgendwelcher Verfahren zur Behandlung des menschlichen oder tierischen Körpers durch Chirurgie oder Therapie und irgendwelcher diagnostischen Verfahren, die am menschlichen oder tierischen Körper vorgenommen werden, bei welchem Meßverfahren Ultraschallsignale in das Medium gesendet und reflektierte Ultraschallsignale von einem Ultraschallsignal-Sende- und -empfangssystem (1; 3, 2, 5) empfangen werden und Charakteristiken des Mediums durch Analyse der empfangenen Signale gemessen werden, welches Meßverfahren die folgenden Schritte umfaßt:

(a) Frequenzanalyse der empfangenen Signale, die von einem Bereich (i) des Mediums (2) empfangen werden, von welchem Bereich die Charakteristiken gemessen werden sollen, um das Energiespektrum von jenen Signalen zu erhalten,

(b) Normieren des bei dem Schritt (a) erhaltenen Energiespektrums mit einem Energiespektrum des Sende- und Empfangssystems als solchem, um die Transferfunktion R(f) des zu untersuchendem Mediums für die empfangenen Signale, welche von dem genannten Bereich reflektiert werden, zu erhalten,

(c) Normieren von R(f) mit einem Wert R(fo) von ihr, der sich auf eine besondere Frequenz fo bezieht, um eine Funktion P(f) zu erhalten,

(d) Ermitteln von P(f)/A(f), wo A(f) ein Ausdruck in einer Funktion Q(f) ist, die durch Normieren einer theoretisch oder empirisch geschätzten Transferfunktion des Mediums für Signale, die von dem genannten Bereich reflektiert wurden, erhalten wird, mit einem Wert der theoretisch oder empirisch ermittelten Transferfunktion, welche sich auf die besondere Frequenz fo bezieht, welche Funktion Q(f) die Form

$$Q(f) = A(f) \cdot e^{B(f)}$$

hat, wobei

A(f) und B(f) Parameter enthalten, deren Werte charakteristisch für den genannten Bereich des Mediums sind,

(e) Ausdrücken von P(f)/A(f), ermittelt bei dem Schritt (d), als ein logarithmischer Exponent, und

(f) Ermitteln von Werten von Mediums-Charakteristik-Parametern, die in den Ausdrücken A(f) und B(f) enthalten sind, durch Berechnung, zum Beispiel durch regressives Verarbeiten mit dem Exponenten, der bei dem Schritt (e) als Funktion einer Frequenz f erhalten wird, der so genommen wird, daß er gleich dem Ausdruck B(f) der theoretisch oder empirisch geschätzten Transferfunktion ist.

2. Meßverfahren nach Anspruch 1, bei dem A(f) eine Funktion der n-ten Potenz ist, wobei n eine Konstante ist, deren Wert charakteristisch für den genannten Bereich ist, und der Frequenz f, und bei dem B(f) eine lineare Funktion der Frequenz f ist, wobei der Koeffizient der Frequenz f in der Funktion auf die Linienintegration der Dämpfungsneigung der Dämpfungskonstanten von Bereichen des Mediums bezogen ist, durch welche Signale von dem genannten Bereichsweg reflektiert werden.

3. Meßverfahren nach Anspruch 1, bei dem B(f) eine quadratische Funktion der Frequenz f, bei der die Koeffizienten des in f linearen Ausdrucks und des in f quadratischen Ausdrucks jeweils auf die Linienintegration der Dämpfungsneigungen der Bereiche des Mediums, durch welche Signale, die von dem genannten Bereich reflektiert werden, hindurchtreten, und den Selbstkorrelationsabstand in der Abtastrichtung bezogen sind.

4. Meßverfahren nach Anspruch 3, bei dem A(f) eine Funktion der vierten Potenz der Frequenz f ist.

5. Meßverfahren nach einem der vorhergehenden Ansprüche, bei dem das Energiespektrum des Sende- und Empfangssystems als solches erhalten wird unter Verwendung eines Standardreflektors in einer Anordnung, in Relation zu dem System, welche der Anordnung des genannten Bereichs des Mediums während der Untersuchung entspricht.

6. Meßverfahren nach einem der vorhergehenden Ansprüche, bei welchen das Medium lebendes Körpergewebe ist.

7. Vorrichtung zum Messen der Charakteristiken eines zu untersuchenden Mediums, mit einem Ultraschallsignalsende- und -empfangssystem (2, 3, 5), das betreibbar ist, um Ultraschallsignale in das Medium zu senden und von dem Medium reflektierte Ultraschallsignale zu empfangen, und Einrichtungen (1, 6, 7, 8, 9) zum Analysieren der empfangenen Signale, um Charakteristiken des Mediums zu messen, mit:—

Frequenzanalyseeinrichtungen (7), die zur Frequenzanalyse der empfangenen Signale betreibbar sind, die von einem Bereich des Mediums reflektiert worden sind, dessen Bereichscharakteristiken gemessen werden sollen, um das Energiespektrum von jenen Signalen zu ermitteln, und

Einrichtungen (8, 9), die betreibbar sind, um das Energiespektrum mit einem Energiespektrum des Sende- und Empfangssystems (2, 3, 5), als solchem zu normieren, um die Transferfunktion R(f) des zu untersuchenden Mediums für die empfangenen Signale, welche von dem genannten Bereich reflektiert wurden, zu erhalten und betreibbar sind, um

R(f) mit einem Wert R(fo) davon zu normieren, welcher sich auf eine besondere Frequenz fo bezieht, um eine Funktion P(f) zu erhalten, und betreibbar sind, um

P(f)/A(f) durch Teilung von P(f) durch A(f) zu erhalten, wobei A(f) ein Ausdruck in einer Funktion Q(f) ist, die durch Normieren einer theoretisch oder empirisch geschätzten Transferfunktion des Mediums für Signale, die von dem genannten Bereich reflektiert wurden, mit einem Wert der theoretisch oder empirisch geschätzten Transferfunktion, der sich auf die besondere Frequenz fo bezieht, erhalten wird, welche Funktion Q(f) die Form

$$Q(f) = A(f) \cdot e^{B(f)}$$

hat, wobei A(f) und B(f) Parameter enthalten, deren Werte charakteristisch für den genannten Bereich des Mediums sind, und betreibbar sind, um

P(f)/A(f) als logarithmischen Exponenten auszudrücken, und betreibbar sind, um

Werte der Mediumscharakteristikparameter, die in den Ausdrücken A(f) und B(f) enthalten sind, durch Berechnung zu erhalten, zum Beispiel durch regressives Verarbeiten mit dem genannten logarithmischen Exponenten, als eine Funktion der Frequenz, der so genommen wird, daß er gleich dem Ausdruck B(f) der theoretisch oder empirisch bestimmten Transferfunktion ist.

8

# 0 100 234

**Revendications**

1. Procédé de mesure de caractéristiques d'un milieu (2) en cours d'investigation, excluant tout procédé de traitement du corps humain ou animal par chirurgie ou thérapie et tout procédé de diagnostic pratiqué sur le corps humain ou animal, procédé de mesure dans lequel des signaux ultrasonores sont émis dans le milieu et des signaux ultrasonores réfléchis sont reçus par un système d'émission et de réception de signaux ultrasonores (1; 3, 2, 5) et des caractéristiques du milieu sont mesurées en analysant les signaux reçus, le procédé de mesure consistant essentiellement:

(a) à analyser en fréquence des signaux reçus, réfléchis par une région (i) du milieu (2), les caractéristiques de cette région devant être mesurées, pour obtenir le spectre de puissance de ces signaux,

(b) à normaliser le spectre de puissance obtenu à la phase (a) avec un spectre de fréquence du système d'émission et de réception lui-mêm pour obtenir la fonction de transfert R(f) du milieu en cours d'investigation pour les signaux reçus, réfléchis par ladite région,

(c) à normaliser R(f) avec l'une de ces valeurs R(fo) concernant une fréquence particulière fo pour obtenir une fonction P(f),

(d) à obtenir P(f)/A(f), où A(f) est un terme d'une fonction Q(f) obtenue en normalisant une fonction de transfert estimée théoriquement ou empiriquement du milieu pour des signaux réfléchis par ladite région avec une valeur de la fonction de transfert estimée théoriquement ou empiriquement concernant la fréquence particulière fo, cette fonction Q(f) ayant la forme

$$Q(f) = A(f) \cdot e^{B(f)}$$

où A(f) et B(f) contenant des paramètres dont les valeurs sont caractéristiques de ladite région du milieu,

(e) à exprimer P(f)/A(f) obtenu à la phase (d) comme un exposant logarithmique, et

(f) à obtenir des valeurs des paramètres caractéristiques du milieu inclus dans les termes A(f) et B(f) par calcul, par exemple par un traitement par régression avec l'exposant obtenu à la phase (e), en fonction de la fréquence, prise à égalité avec le terme B(f) de la fonction de transfert estimée théoriquement ou empiriquement.

2. Procédé de mesure selon la revendication 1, dans lequel A(f) est une fonction d'une puissance n, où n est une constante, dont la valeur est caractéristique de ladite région et a la fréquence f et dans lequel B(f) est une fonction linéaire de la fréquence f, le coefficient de la fréquence dans la fonction étant lié à l'intégration linéaire des pentes d'atténuation des constantes d'atténuation des régions du milieu par lesquelles passent les signaux réfléchis par ladite région.

3. Procédé de mesure selon la revendication 1, dans lequel B(f) est une fonction quadratique de la fréquence f, dans lequel les coefficients du terme linéaire en f et le terme quadratique en f sont liés respectivement à l'intégration linéaire des pentes d'atténuation des régions du milieu par lesquelles passent les signaux réfléchis par ladite région et de la distance d'auto-corrélation dans la direction de balayage.

4. Procédé de mesure selon la revendication 3, dans lequel A(f) est une fonction de la quatrième puissance de la fréquence f.

5. Procédé de mesure selon l'une quelconque des revendications précédentes, dans lequel le spectre de puissance du système d'émission et de réception en lui-même est obtenu en utilisant un réflecteur standard dans une disposition, en relation avec le système, correspondant à la disposition de ladite région du milieu en cours d'investigation.

6. Procédé de mesure selon l'une quelconque des revendications précédentes, dans lequel le milieu est un tissu de corps vivant.

7. Appareil de mesure des caractéristiques d'un milieu en cours d'investigation, comportant:

un système d'émission et de réception de signaux ultrasonores (2, 3, 5) ayant pour fonction d'émettre des signaux ultrasonores dans le milieu et de recevoir des signaux ultrasonores réfléchis par le milieu, et

un dispositif (1, 6, 7, 8, 9) destiné à analyser les signaux reçus pour mesurer des caractéristiques du milieu, comportant:

un dispositif analyseur de fréquence (7) ayant pour fonction d'analyser en fréquence les signaux reçus réfléchis par une région du milieu, région dont les caractéristiques doivent être mesurées pour obtenir le spectre de puissance de ces signaux, et

un dispositif (8, 9) ayant pour fonction de normaliser le spectre de puissance avec un spectre de puissance du système d'émission et de réception (2, 3, 5) en lui-même pour obtenir la fonction de transfert R(f) du milieu en cours d'investigation pour les signaux reçus réfléchis par ladite région, ayant également pour fonction de normaliser R(f) avec une de ces valeurs R(fo) concernant une fréquence particulière fo pour obtenir une fonction P(f), et ayant aussi pour fonction:

d'obtenir P(f)/A(f) en divisant P(f)/A(f), où A(f) est un terme d'un fonction Q(f) obtenue en normalisant une fonction de transfert estimée théoriquement ou empiriquement du milieu pour des signaux réfléchis par ladite région avec une valeur de la fonction de transfert estimée théoriquement ou empiriquement concernant la fréquence particulière fo, cette fonction Q(f) ayant la forme

$$Q(f) = A(f) \cdot e^{B(f)}$$

où A(f) et B(f) contiennent des paramètres dont les valeurs sont caractéristiques de ladite région du milieu et ayant pour fonction:

d'exprimer P(f)/A(f) comme un exposant logarithmique et ayant pour aussi pour fonction d'obtenir des valeurs de paramètres caractéristiques du milieu, inclus dans les termes A(f) et B(f) par calcul, par exemple par traitement par régression avec ledit exposant logarithmique, comme une fonction de la fréquence, prise égale au terme B(f) de la fonction de transfert estimée théoriquement ou empiriquement.

0 100 234

Fig. 1

Fig. 2

Fig. 3

Fig. 4